# EUROPEAN PATENT APPLICATION

(11) **EP 2 402 028 A1**
(43) Date of publication of application: **04.01.2012**
(21) Application number: 10305717.0
(22) Date of filing: 01.07.2010
(51) Int. Cl.: A61K 39/125, C07K 14/085, A61P 31/14

(54) **Enterovirus vaccines for preventing and treating type 1 diabetes (II)**

(71) Applicant: Sanofi Pasteur, 69007 Lyon Cedex (FR); Vactech OY, 33520 Tampere (FI)
(72) Inventor: Hyöty, Heikki, 33230, TAMPERE (FI); Knip, Mikael, 00130, HELSINKI (FR); Laitinen, Olli, 33270, TAMPERE (FI); Tolonen, Outi, 90120, OULU (FI); Pulkki, Minna, 33560, TAMPERE (FI); Oikarinen, Sami, 33500, TAMPERE (FI); Honkanen, Hanna-Riikka, 33520, TAMPERE (FI); Lecouturier, Valérie, 69380, CHAZAY d'AZERGUES (FR); Almond, Jeffrey, 69570, DARDILLY (FR)
(74) Representative: Boije-Backman, Solveig Magdalena

(57) **Abstract**

New enteroviruses associated with type 1 diabetes have been found, which opens up new therapeutic and diagnostic implications. A vaccine against at least one enterovirus selected from coxsackie A virus CAV4, CAV5, CAV6 and echovirus 18 is provided for use in preventing or treating type 1 diabetes. A diagnostic assay for predicting the risk of contracting type 1 diabetes based on the determination of said enteroviruses is also provided.

## Description

### Field of the Invention

The present invention relates to vaccines for preventing or treating type 1 diabetes. Especially the invention relates to a vaccine comprising at least one enterovirus, a component thereof or an antibody thereto, wherein the virus is selected from a group strongly associated with the risk of contracting type 1 diabetes. A diagnostic assay for predicting the risk of contracting the disease.

### Background of the invention

Type 1 diabetes is a severe disease that has become more and more frequent already at a very early age. In type 1 diabetes the beta-cells of the pancreas are destroyed, which leads to insulin deficiency. The destruction of the beta-cells is believed to be caused by an autoimmune response, which in turn is assumed to be induced by a virus infection.

The connection between enteroviruses and type 1 diabetes (T1D) has been documented in a multitude of studies. Enteroviruses have been detected in the pancreas, blood and intestinal mucosa of patients with type 1 diabetes more frequently than in control subjects and prospective studies have supported their role in the initiation of the beta-cell damaging process associated with type 1 diabetes. Enteroviruses infect pancreatic beta-cells in cell culture and cause diabetes in animal models. Moreover, recent studies indicate that the genetic susceptibility to type 1 diabetes is modulated by a polymorphism in the *IFlH1* gene which encodes an innate immune system receptor for enteroviruses. This evidence has generated increasing demands to develop vaccines against diabetogenic enteroviruses. However, it is not known which enterovirus serotypes are involved and their identification would be needed for the development of effective vaccines. Previous studies have suggested that coxsackie B serotypes may be important, but still the knowledge of which enterovirus serotypes are involved in the disease is limited.

The group of enteroviruses includes more than 100 different serotypes, and because a vaccine covering all the 100 enteroviruses is not realistic using the current standard vaccine technologies, the knowledge of which serotypes are involved in type 1 diabetes is critical for vaccine development. Enterovirus infections are usually subclinical, but they may also cause various kinds of diseases. For example polioviruses, coxsackie B virus (CBV), coxsackie A virus (CAV), and echovirus (E, or Echo) as well as numbered enteroviruses are enteroviruses known to be involved in the development of a variety of diseases. The spectrum of responsible serotypes varies a lot from disease to disease, and even in one disease like type 1 diabetes the exact serotypes have not been fully and reliably identified. Indeed, previous studies reporting association between enteroviruses and T1D were not able to discriminate between serotype or were restricted to case reports. Some studies have suggested that CBV serotypes may be important (Yoon et al, 1979 New Eng J of Med, v300, p1173; Hindersson M, et al., 2005, J Clin Virol v.33 p158; Dotta F. et al., 2007, PNAS 104: 5115). However, other enterovirus serotypes have also been sporadically reported (Cabrera-Rode et al., 2003, Diabetologia; Wil-liams et al., 2006, J Clin Micro v.44 p441).

WO01/00236 (Hyöty et al.) suggests the use of oral poliovirus in a vaccine against type 1 diabetes. In addition it suggests another diabetes vaccine comprising one or more inactivated non-polio enteroviruses selected from the group consisting of CBV serotypes 1, 2, 3, 4, 5 and 6, echovirus serotypes 3, 4, 6, 9, 11, 22 and 30, CAV serotypes 9 and 16. However, no data is given as to the role of the individual serotypes listed, except for some animal tests with CBV3. A more recent publication US2010/0047273 (Rappuoli et al.) suggests the use of CBV4, and in particular of a Tuscany strain thereof for preventing or treating type 1 diabetes.

Still there is a great need for developing effective means and methods for preventing and treating type 1 diabetes. The present invention meets this need.

### Summary of the Invention

The present invention resides in the finding of new enterovirus serotypes that have not been associated with type 1 diabetes previously. The new serotypes open new therapeutic implications in the prevention and treatment of type 1 diabetes.

The present invention now provides a vaccine comprising at least one enterovirus selected from the group consisting of: coxsackie A virus CAV4, CAV5, CAV6 and echovirus E18, or a component thereof.

The invention also provides a vaccine comprising an antibody against at least one enterovirus selected from the group consisting of: coxsackie A virus CAV4, CAV5, CAV6 and echovirus E18, or against a component thereof.

The invention still further provides these vaccines for use in preventing or treating type 1 diabetes.

In addition the invention provides a diagnostic assay for predicting the risk of contracting type 1 diabetes comprising determining the presence of at least one enterovirus selected from the group consisting of: coxsackie A virus CAV4, CAV5, CAV6 and echovirus E18, a component thereof, or an antibody thereto in a sample from a subject.

Further herein is disclosed a method of preventing or treating type 1 diabetes in a subject in need thereof, comprising vaccinating the subject with at least one enterovirus selected from the group consisting of: coxsackie A virus CAV4, CAV5, CAV6 and echovirus E18, a component thereof, or an antibody thereto.

Some preferred embodiment of the invention are set forth in the dependent claims.

### Brief Description of the Drawings

Figure 1 shows the ratio of enterovirus infection and samples monthly.
Figure 2 shows the duration of virus excretion.
Figure 3 shows the ratio of enterovirus infections and samples in age classes.
Figure 4 shows the ratio of all enterovirus infections and samples (upper panel), and infections and samples before the appearance of autoantibodies (AAB) (lower panel).

### Detailed Description of the Invention

The disclosed vaccine comprises at least one enterovirus selected from the group consisting of coxsackie A virus CAV4, CAV5, CAV6 and echovirus E18, or a component thereof in a pharmaceutically effective amount.

A "pharmaceutically effective amount" of the vaccine is an amount, which is able to elicit or mediate an immune response that protects the vaccine recipient against the enterovirus for example by inducing neutralizing antibodies.

The enterovirus is preferably inactivated, or the vaccine contains only a component of the virus. The "component" may be an immunogenic structure of the virus such as a subunit thereof, or a nucleic acid fragment such as part of the genome of the virus. The component may also be synthetically produced or modified. Thus the vaccine may include whole viruses, the infectivity of which has been inactivated, or subunit vaccines containing certain antigenic structures, proteins or peptides of the virus, or their combination (such as virus like particles), or fragments of viral RNA or cDNA encoding the whole virus or individual viral proteins or inactivated forms of the virus. Inactivated vaccines may be produced by propagating the virus in cell cultures and by purifying it from infected cells and culture media by high-speed centrifugation in a density gradient formed by sucrose or other high-density media. Alternatively the virus may be purified by chromatography. The infectivity of the purified viruses is destroyed by inactivating the viruses by chemical treatment (e.g. formalin inactivation like that used to produce inactivated polio virus vaccine), irradiation or heat treatment. Subunit vaccines may consist of purified viral proteins or recombinant viral proteins, synthetic peptides corresponding to viral antigenic epitopes, virus like particles or empty viral capsids, which are produced during infection but lack the viral genome. These subunit vaccines can be administered either as such or conjugated to haptens or carriers (e.g. ISCOM particles, chitosan, TLR agonists, biodegradable microparticles).

Alternatively the vaccine comprises an antibody against at least one of said enteroviruses in a pharmaceutically effective amount. This antibody may be raised against the whole virus or against an immunogenic component thereof, or it may be recombinantly produced. The term antibody as used herein includes fragments of antibodies such as Fab-fragments and scFv fragments.

The above mentioned vaccines can be given parenterally by injections, perorally, intradermally, transcutaneously, sublingually, intranasally, as inhalation, or per rectum. Each immunizing dose includes viral structures in a titer, which is able to induce proper immune response in humans. This dose would correspond to that used in Salk-type inactivated poliovirus vaccine including 1.8 - 2 µg of viral protein per each dose and 20 - 40 antigenic D-units of poliovirus type 1, 4 - 8 antigenic D-units of poliovirus type 2 and 16 - 32 antigenic D-units of poliovirus type 3. The dose may also be another, if it has been confirmed to be safe and immunogenic or able to stimulate the immune system.

The enteroviruses, their components or antibodies can be given in different combinations including one or more enterovirus serotypes given simultaneously or serially. If the different enteroviruses, their components or antibodies are given simultaneously the pharmaceutical composition may be in the form of a mixture of different enteroviruses, components or antibodies. Preferably the vaccine contains a cocktail of diabetogenic enterovirus serotypes selected from the group consisting of CAV4, CAV5, CAV6 and E18. Preferably it further contains at least one or more of the serotypes selected from the group consisting of CBV1, CBV2 and CAV16. According to particular embodiments the vaccine comprises a mixture of at least the following serotypes:
CAV16, CAV4, CAV5, CAV6 and E18,
CBV2, CAV4, CAV5, CAV6 and E18,
CBV2, CAV16, CAV4, CAV5, CAV6 and E18, or
CBV1, CBV2, CAV16, CAV4, CAV5, CAV6 and E18.

The serotypes of all the described mixtures may also be administered separately or in any possible combination.

The enterovirus, component or antibody is formulated into a pharmaceutical composition comprising a pharmaceutically effective amount of the virus, its component or antibody. In addition to the active ingredients that elicit immune stimulation the compositions may further comprise pharmaceutically acceptable excipients, carriers, haptens and adjuvants. Excipients, carriers, haptens and adjuvants may include for example phenoxyethanol, magnesium chloride, sucrose, thiomersal, formaldehyde, phenol, antibiotics (preservatives) or aluminium salts, polymer microparticles, ISCOM particles, carrier proteins (e.g. cholera toxin), liposomes, protein micelles (haptens/adjuvants), TLR agonists or other activators of the innate immune system.

The pharmaceutical composition is preferably administered to a child within 5 years after birth, and more preferably within 3 years, especially within 2 years, and most preferably within 1 year after birth, with boosters given later in life to prevent or treat type 1 diabetes. It can for example be given at the age or 3, 6 or 12 months, with boosters at older age. It can also be given to pregnant mothers to prevent type-1-diabetes in the baby, or prenatally to the pregnant mother and postnatally to the baby. Vaccine-induced antibody response of the mother protects the child because IgG class maternal antibodies are transferred to the fetus through the placenta and are thus protecting the child until the age of 6-12 months when maternal antibodies disappear from the child's circulation.

The vaccination regime can be used in the whole population or in a subpopulation carrying increased risk for presenting with type 1 diabetes. Such high-risk groups may include mothers or children with HLA-conferred genetic risk (HLA DR3 and/or DR4) subjects with type 1 diabetes in first- or second-degree relatives or children tetsing positive for two or more diabetes-associated autoantibodies.

The finding of new enterovirus serotypes associated with induction of type 1 diabetes is also useful in a diagnostic assay for predicting the risk of contracting the disease by determining the presence of at least one of the enterovirus serotypes CAV4, CAV5, CAV6 or E18. The presence of the virus may be determined e.g. in a stool sample by PCR, or antibodies against the virus may be determined in a blood sample taken from the subject to be tested e.g. by a plaque inhibition assay or microneutralizaton analysis of the serum or plasma sample. as described in the example. In addition, the virus may be detected in various tissues using e.g. RT-PCR, in situ hybridization or immuno-histochemistry.

The vaccines described may be used in preventing and treating type 1 diabetes, in inducing an immune response against a diabetogenic enterovirus, and in eliminating the diabetogenic effect of an enterovirus in a subject in need thereof by vaccinating the subject with at least one enterovirus selected from the group consisting of: coxsackie A virus CAV4, CAV5, CAV6 and echovirus E18, a component thereof, or an antibody thereto.

### Example

### Stool analyses

Altogether, 4184 stool samples from 359 children (102 cases and 257 controls) have been screened for enterovirus (EV) positivity. In Table 1 all these samples mentioned above have been divided into different categories. In EV screening PCR in total 230 separate infections were identified out of which 166 could be serotyped by sequencing.

**Table 1. EV-screened stool samples divided into different categories.**

| | **Whole data** | **Case** | **Control** |
|---|---|---|---|
| Number of groups | 102 | | |
| Number of children | 359 | 102 | 257 |
| Number of stool samples | 4184 | 1321 | 2863 |
| Number of enterovirus positive stool | 307 | 103 | 204 |
| samples | | | |
| Amount of separate infections | 230 | 75 | 155 |
| Number of successfully serotyped infections | 166 (72%) | 54 (72%) | 112 (72%) |
| Number of untyped infections | 64 (28%) | 21 (28%) | 43 (28%) |
| Number of different serotypes | 28 | 17 | 24 |

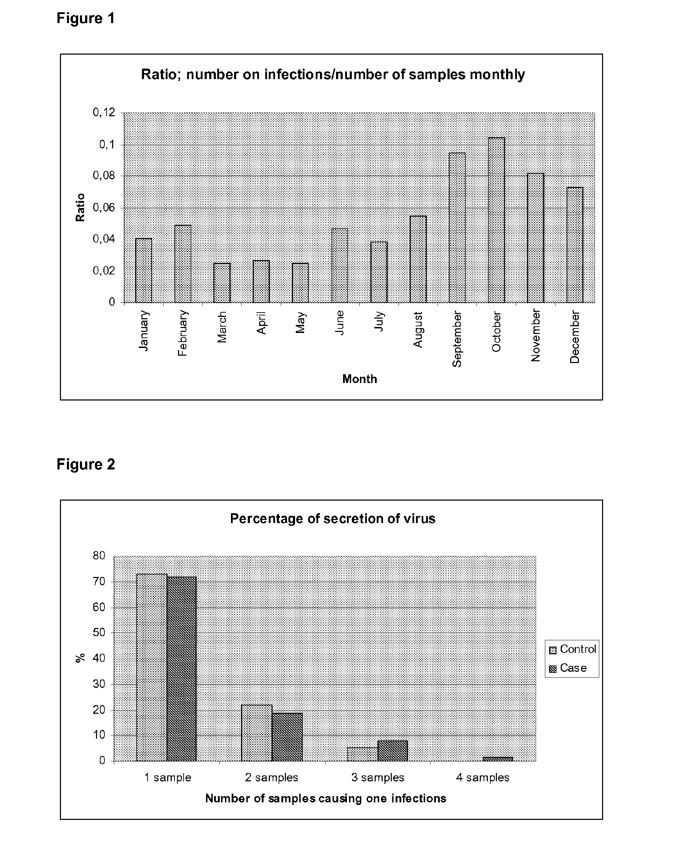

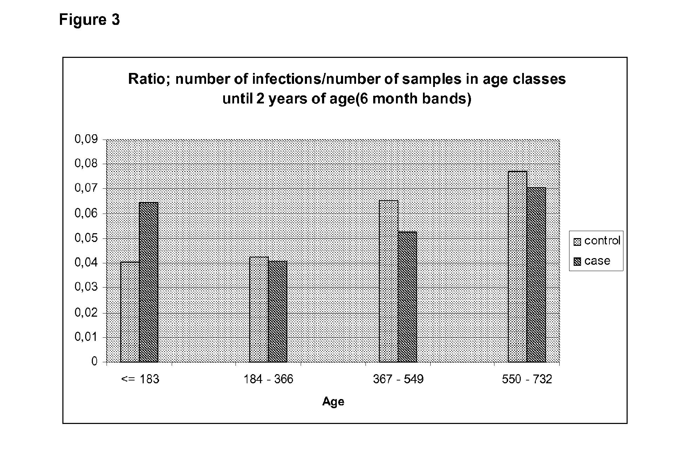

The annual distribution of identified infections, the duration of infections and the ratio of infections and samples in age classes are presented in Figures 1, 2 and 3, respectively as follows: The ratio of infections and samples monthly is shown in Figure 1, where the ratio is calculated as number of infections monthly/number of samples monthly. The duration of virus excretion is shown in Figure 2 as percentage of the number of serial samples positive for the same virus strain. The ratio of infections and samples in age classes (six months bands) until two years of age is shown in Figure 3. The ratio is calculated as number of infections in age class/number of all samples in the age class.

The strictly defined criteria were set for case and control children, according to which, for example, the difference in the day of birth between case child and control child was defined to be in maximum ± 2 months. In practice, in six case/control groups the difference of one control children was outside the defined criteria. However, of these in 5 cases the difference was only 64, 64, 65, 69 and 80 days making the deviation very small. Only in the case of one case/control group the deviation for one control child was marked the difference being 271 days. In addition, two control children turned type 1 diabetic during the follow up period.

### Serotyping the enteroviruses detected from stool samples in the whole cohort

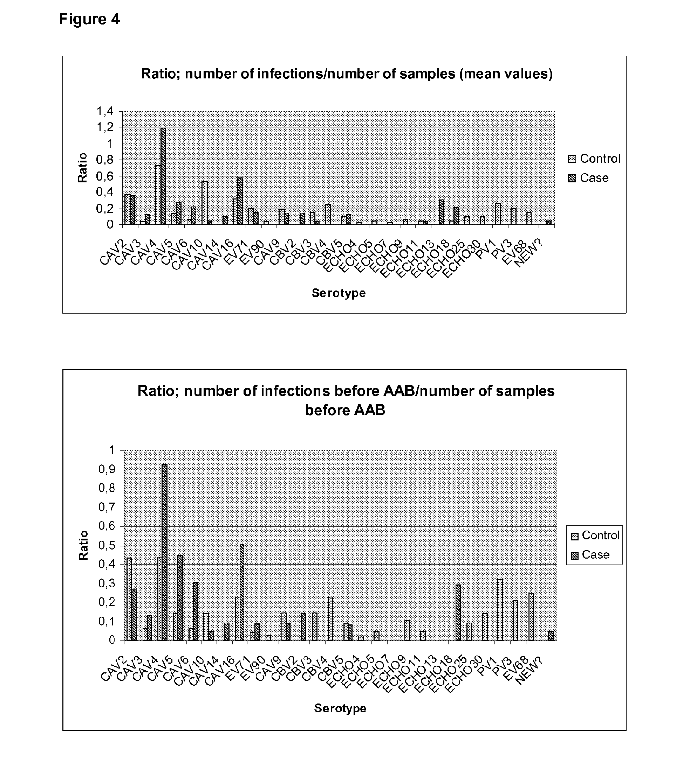

From all (307) enterovirus positive stool samples the serotype has been identified by VP1/VP2 sequencing from 166 infections (infection=same serotype in one or in two or more consecutive samples). Figure 4 shows the proportions of all identified serotypes presented as a ratio of infections and samples collected. In the upper panel the ratio of all the infections and samples is calculated as number of infections/number of samples, and in the lower panel the ratio of the infections and samples before the appearance of autoantibodies AAB is calculated as number of infections before ABB/number of samples before AAB. The ratio of different serotypes per samples collected is first calculated for every case child and every control child, here presented as mean value of all cases and all controls.

According to the above data, three kinds of serotypes can be seen. One group includes serotypes which are more common in case than in control children, the second group comprises serotypes which are more frequent in control than in case children, and the third group those which are about equally common in both cases and controls. Based on this we formed the following groups of viruses: serotypes CAV4, CAV5, CAV6, CAV16 and ECHO 18 were chosen to be diabetogenic group 1; and serotypes CAV4, CAV5, CAV6, CBV2 and ECHO18 as diabetogenic group 2. When the frequency of infections by the risk group serotypes was analyzed as a group a statistically significant risk-effect was observed (Table 2). These analyses addressed the question if one or more infections by any of the risk group serotypes (vs. having none of these risk group serotypes) has a statistically significant effect.

High OR was received when comparing males and females separately. Before the appearance of autoantibodies, females had the highest OR (OR=6.48, P = 0.008). In contrast, males had the highest OR within the 12-month window before the before seroconversion to autoantibody positivity (OR=8.49, P = 0.008) (two risk groups combined). The highest OR was received one year before AAB in those children that had the risk HLA genotype (OR=9.86, P=0.004) (two risk groups combined). The same trend was also seen in diabetic cases and their controls; the highest ORs were received before and one year before seroconversion to autoantibodies (Table 3).

**Table 2. Statistical analysis of different groups of serotypes; two risk groups, and two risk groups combined.**

| **case** | **Odds Ration** | **P>\|z\|** | **95% lower limit** | **95% upper limit** |
|---|---|---|---|---|
| **Risk group 1** | | | | |
| **Whole cohort** | | | | |
| CAV4, CAV5, CAV6, CAV16, ECHO18 | **1.92** | 0**.030** | | **3.47** |
| **Whole cohort before AAB** | | | | |
| CAV4, CAV5, CAV6, CAV16, ECHO18 | **2.69** | | | **5.39** |
| **Whole cohort one year be-** | | | | |
| **fore AAB** | | | | |
| CAV4, CAV5, CAV6, CAV16, ECHO18 | **3.72** | | | **10.21** |

| **Risk group 2** | | | | |
|---|---|---|---|---|
| **Whole cohort** | | | | |
| CAV4, CAV5, CAV6, CBV2, ECHO18 | **2.39** | | | **4.57** |
| **Whole cohort before AAB** | | | | |
| CAV4, CAV5, CAV6, CBV2, ECHO18 | **3.16** | | | **6.84** |
| **Whole cohort year before** | | | | |
| AAB | | | | |
| CAV4, CAV5, CAV6, CBV2, ECHO18 | **6.53** | | | **20.60** |

| **Risk groups 1 and 2 combined** | | | | |
|---|---|---|---|---|
| **Whole cohort** | | | | |
| CAV4, CAV5, CAV6, CAV16, | | | | |
| CBV2, ECHO18 | **1.92** | | | **3.47** |
| **Whole cohort before AAB** | | | | |
| CAV4, CAV5, CAV6, CAV16, | | | | |
| CBV2, ECHO18 | **2.91** | | | **5.84** |
| **Whole cohort year before** | | | | |
| **AAB** | | | | |
| CAV4, CAV5, CAV6, CAV16, | | | | |
| CBV2, ECHO18 | **7.30** | **0.001** | **2.32** | **22.89** |

**Table 3. Statistical analysis of different groups of serotypes; two risk groups. Only cases, which have T1 D and their controls.**

| **ONLY TYPE 1 DIABETES (T1D) CASES AND** | | | | |
|---|---|---|---|---|
| **THEIR CONTROLS** | | | **102 cases** | **63 T1 D cases*** |
| **Case** | **Odds Ration** | **P>\|z\|** | **95% lower limit** | **95% upper limit** |
| **Risk group 1** | | | | |
| **Whole cohort** | | | | **4.85** |
| CAV4 CAV5, CAV6, CAV16, | | | | |
| ECHO18 | **2.31** | **0.028** | **1.09** | |
| **Whole cohort before AAB** | | | | |
| CAV4 CAV5, CAV6, CAV16, | | | | |
| ECHO18 | **4.49** | **0.003** | **1.67** | **12.07** |
| **Whole cohort year before** | | | | |
| **AAB** | | | | |
| CAV4 CAV5, CAV6, CAV16, | | | | |
| ECHO18 | **3.99** | **0.029** | **1.14** | **13.87** |

| **Risk group 2** | | | | |
|---|---|---|---|---|
| **Whole cohort** | | | | |
| CAV4 CAV5, CAV6, CBV2, | | | | |
| ECHO18 | **2.91** | **0.014** | **1.24** | **6.84** |
| **Whole cohort before AAB** | | | | |
| CAV4 CAV5, CAV6, CBV2, | | | | |
| ECHO18 | **5.58** | **0.004** | **1.73** | **18.05** |
| **Whole cohort year before** | | | | |
| **AAB** | | | | |
| CAV4 CAV5, CAV6, CBV2, | | | | |
| ECHO18 | **5.04** | **0.02** | **1.28** | **19.78** |

The number of children and identified infections in combined risk groups 1 and 2 are presented in Table 4.

**Table 4. Number of children and infections in combined risk groups 1 and 2.**

| | | | **Number of children** | | **Number of infections** | |
|---|---|---|---|---|---|---|
| | | | **Case** | **Control** | **Case** | **Control** |
| **Risk groups** | **1** | **Whole cohort** | **25** | **39** | **33** | **45** |
| **and** | **2** | **Before AAB** | **21** | **21** | **25** | **29** |
| **combined** | | **Year Before AAB** | **13** | **6** | **13** | **13** |

It seems that in the stool analysis the risk effect of certain coxsackie A viruses is seen if the infection is suffered in very young age and especially if child has the moderate risk HLA genotype. In addition they might be associated with an aggressive autoimmune process which leads to severe beta cell damage and diabetes.

In conclusion, when several serotypes were combined, a group of risk viruses could be recognized. CAV4, CAV5, CAV6, CAV16 and E18 are the most interesting as potential risk viruses. It is remarkable that CAV4, CAV5, CAV6 and CAV16 are genetically closely related to each other. The clearest risk effect was associated with male gender in the restricted time window before the autoantibody seroconversion date.

## Claims

1. A vaccine comprising at least one enterovirus selected from the group consisting of: coxsackie A virus CAV4, CAV5, CAV6 and echovirus E18, or a component thereof.

2. The vaccine of claim 1, wherein the enterovirus is an inactivated virus.

3. The vaccine of claim 1, wherein the enterovirus component is a subunit or a nucleic acid of said enterovirus.

4. The vaccine of any one of the preceding claims, further comprising at least one enterovirus of serotype coxsackie B virus CBV1, CBV2 or coxsackie A virus CAV16, or a component thereof.

5. The vaccine of any one of claims 1-4, comprising a virus or a component of each of the following enterovirus serotypes: CAV16, CAV4, CAV5, CAV6 and E18.

6. The vaccine of any one of claims 1-4, comprising a virus or a component of each of the following enterovirus serotypes: CBV2, CAV4, CAV5, CAV6 and E18.

7. The vaccine of any one of claims 1-4, comprising a virus or a component of each of the following enterovirus serotypes: CBV2, CAV16, CAV4, CAV5, CAV6 and E18.

8. The vaccine of any one of claims 1-4, comprising a virus or a component of each of the following enterovirus serotypes: CBV1, CBV2, CAV16, CAV4, CAV5, CAV6 and E18.

9. The vaccine of any one of the previous claims for use in preventing or treating type 1 diabetes.

10. The vaccine of claim 9, wherein the use comprises administering it to a pregnant woman, and optionally postnatally to her baby.

11. A vaccine comprising an antibody against at least one enterovirus selected from the group consisting of: coxsackie A virus CAV4, CAV5, CAV6 and echovirus E18, or against a component thereof.

12. The vaccine of claim 11 for use in preventing or treating type 1 diabetes.

13. Diagnostic assay for predicting the risk of contracting type 1 diabetes comprising determining the presence of at least one enterovirus selected from the group consisting of: coxsackie A virus CAV4, CAV5, CAV6 and echovirus E18, a component thereof, or an antibody thereto in a sample from a subject.

14. Method of preventing or treating type 1 diabetes in a subject in need thereof, comprising vaccinating the subject with at least one enterovirus selected from the group consisting of: coxsackie A virus CAV4, CAV5, CAV6 and echovirus E18, a component thereof, or an antibody thereto.
